# EUROPEAN PATENT SPECIFICATION

(11) **EP 2 453 873 B1**
(45) Date of publication and mention of the grant of the patent: **30.06.2021**
(21) Application number: 10737362.3
(22) Date of filing: 16.07.2010
(51) Int. Cl.: A61K 9/00, A61K 47/10

(54) **IMPROVEMENTS IN AND RELATING TO COLON CLEANSING COMPOSITIONS**
VERBESSERUNGEN BEI UND IM ZUSAMMENHANG MIT DICKDARMREINIGUNGSZUSAMMENSETZUNGEN
AMÉLIORATIONS APPORTÉES À DES COMPOSITIONS DE NETTOYAGE DU COLON OU ASSOCIÉES À DE TELLES COMPOSTIONS

(30) Priority: 17.07.2009 GB 0912487; 18.02.2010 US 305814 P
(43) Date of publication of application: 23.05.2012
(73) Proprietor: Norgine BV, 1083 HP Amsterdam (NL)
(72) Inventor: ATTWELL, Mark, Mid-Glamorgan CF82 8SJ (GB); COCKETT, Alasdair, Mid-Glamorgan CF82 8SJ (GB); COX, Ian, Mid-Glamorgan CF82 8SJ (GB); GRANGER, Janet, Mid-Glamorgan CF82 8SJ (GB); OZA, Pankaj, Mid-Glamorgan CF82 8SJ (GB); PADFIELD, Dawn, Mid-Glamorgan CF82 8SJ (GB); PADFIELD, Nick, Mid-Glamorgan CF82 8SJ (GB)
(74) Representative: Abel & Imray
(86) International application number: PCT/GB2010/001362
(87) International publication number: WO 2011/007153

(56) References cited:
- WO-A1-2004/037292
- WO-A1-2009/055846
- WO-A2-2009/052256
- Anonymous: "Summary of product characteristics: Laxido Orange, powder for oral solution"[Online] 10 November 2008 (2008-11-10), pages 1-5, XP002598513 Galen Retrieved from the Internet: URL:http://www.galen.co.uk/files/Laxido%20 Orange%20ROI%20SPC%20sugary.pdf> [retrieved on 2010-08-20]
- None

## Description

The present invention relates to colon cleansing compositions for cleansing the gastrointestinal tract. Colon cleansing compositions are also known as lavage solutions, bowel preparations or colonic evacuants.

Colon cleansing is important before numerous surgical or diagnostic procedures, including colonoscopy, barium enema examination, sigmoidoscopy and colon surgery. Such procedures are often carried out on an outpatient basis and thus it is desirable that the colon cleansing be carried out by the patient at home, prior to arrival at the hospital or surgery where the procedure is to take place. It is therefore important that patient compliance is good without medical supervision for satisfactory colon cleansing to be achieved prior to the procedure.

Intestinal lavage, in which a large volume of an electrolyte solution containing sodium sulphate and polyethylene glycol (PEG) is ingested, is one of the most common methods for colon cleansing. These osmotically active agents are nonabsorbable or only poorly absorbable and thus retain water in the bowel, resulting in copious diarrhoea and cleansing of the colon. Whilst effective, such compositions have a very salty taste that adversely affects patient compliance. Various attempts have been made to improve the taste of colon cleansing compositions to improve patient compliance, by reducing sodium and sulphate content or by adding flavours to mask saltiness and make the compositions more palatable. It has, however, proved difficult to adequately reduce or mask the very unpleasant salty taste of such compositions whilst maintaining effective amounts of the osmotic salts.

Moreover, for effective cleansing, many of these compositions must be ingested in quantities of between 2 to 4 litres. The unpleasant taste of these compositions combined with the large volumes required to be ingested often contributes to nausea or vomiting, resulting in poor patient compliance and failure to consume the full volume of solution.

A number of improved colon cleansing compositions are described in WO 2004/037292. The compositions described therein are effective despite being taken in a lower volume than other colon cleansing solutions. Typically, only 2 litres of the solution need to be taken by the patient. A colon cleansing composition according to WO 2004/037292 that comprises PEG, sodium sulphate, an ascorbate component and lemon flavouring is commercialised under the tradename MOVIPREP® (registered trademark of Velinor AG, a member of the Norgine group of companies). In the MOVIPREP product, PEG 3350, sodium sulphate, sodium chloride, potassium chloride, sweetener and lemon flavouring are provided in a sachet A, and the ascorbate component (comprising ascorbic acid and sodium ascorbate) is provided in a sachet B.

Despite the success of the lemon flavoured MOVIPREP composition, there is always room for improvement in the taste, thereby reducing still further one of the major obstacles to patient compliance. Improving the taste of colon cleansing compositions containing organic acids or salts thereof, for example the ascorbate component in the MOVIPREP composition, presents a particular challenge in view of the taste effects contributed by the organic acids themselves. It is difficult to mask the saltiness of colon cleansing compositions without making the compositions excessively sweet.

An important consideration for the viability of any medical product is the shelf-life and stability of the product over time. It must be possible for medical products to be stored and/or distributed over prolonged times, typically at least one year, more usually 2 or 3 years from their date of manufacture. There are several criteria that must be considered when assessing a product's stability and shelf-life: it is important that the product remains sterile for the whole of its shelf-life. It is also important that it remains effective over the whole of its shelf-life, including that its taste does not deteriorate. In compositions that include flavourings, that includes that the flavouring intensity should not decline significantly over the shelf-life time. Finally, the physical appearance and the physical properties should not alter significantly over the shelf-life time; that includes that a powder should remain flowable and should not aggregate in time. It should also not become discoloured to a significant extent. There is a risk that aggregates or lumps that appear in a powder on storage are not properly dissolved when a patient prepares a solution with the powder. Incomplete dissolution may result in a solution that contains its constituents in incorrect amounts, and such a solution may have impaired efficacy and/or taste.

It has been known for certain medical products containing orange flavourings to become lumpy or for the orange taste to decrease to below the desired level in 6 month stability tests such that only short shelf-lives are achieved. This stability problem is a particular issue for products comprising polyethylene glycol and electrolytes. For example, Laxido Orange is a constipation treatment marketed by Galen Limited (see http://www.galen.co.uk) that contains 13.125g PEG 3350, 350.7mg sodium chloride, 178.5mg sodium hydrogen carbonate, 46.6mg potassium chloride, acesulfame potassium and orange flavouring (containing glucose), and is supplied as a dry powder. For use, the powder is made up to 125ml with water. It has been found that samples of Laxido Orange dry powder do not perform well in 3 and 6 month stability tests. In samples (in their sachet packaging as sold) stored at 25°C and 60% relative humidity, and in samples stored at 40°C and 75% relative humidity, for 3 or 6 months, the originally white powder becomes off-white and develops small lumps. The person skilled in the art would thus be deterred from developing orange flavoured compositions of PEG and electrolytes Further, WO 2004/037292 A1 also mentions the use of a dry composition for colon cleansing comprising lemon flavour.

Surprisingly, it has now been found by the current inventors that when the flavouring in sachet A of the MOVIPREP composition is orange (instead of lemon), despite the fact that this comprises a mixture of PEG and electrolytes, the composition of sachet A passes all physical stability tests over 24 months, including, for example, remaining free-flowing and substantially free from lumps. It has also been found that solutions prepared from the contents of sachets A and B of the MOVIPREP product in which the flavouring in sachet A is orange (instead of lemon) are stable over a period of 24 hours in solution stability tests, including after storage for 12 months.

The current inventors have found that orange flavoured compositions have improved taste and acceptable storage stability.

Accordingly, the present invention provides a dry composition for admixture with water, comprising, per litre of solution to be made, the following components:
(a) 85 to 115 g polyethylene glycol (PEG) having an average molecular weight of 2500 to 4500 Da;
(b) 6 to 9 g sodium sulphate;
(c) 2 to 3 g sodium chloride;
(d) 0.5 to 1.5 g potassium chloride;
(e) 5 to 15 g of an organic acid component; and
(f) orange flavouring comprising maltodextrin in an amount of 70 to 90% by weight of the flavouring.

"Dry" in this context means that the composition has no added liquid, especially no added water. For example, it is substantially free from water. Small amounts of water or other liquid may be present in some of the components even when they are supplied as "dry", for example a salt may be present as a hydrate. Such amounts of water are not considered substantial. "Solution" in this context includes any mixture resulting from admixture of the composition with water, whether fully dissolved or not. The composition is optionally presented in two or more parts. For example, it may be presented as a part A comprising the components (a) to (d) and (f), and a part B comprising the component (e). Component (f) may optionally be provided in a third part (part C) instead of or as well as being in part A.

The orange flavouring in compositions of the invention comprises maltodextrin. Solutions of compositions of the invention comprising flavouring including maltodextrin as one of its components were surprisingly found to have particularly acceptable taste. Preferably maltodextrin is present in the flavouring in an amount of 70 to 80% by weight of the flavouring. It has been found that a solution comprising flavouring in which maltodextrin is present in an amount of less than 80% by weight of the flavouring was preferred over solutions containing more maltodextrin. Preferably, the maltodextrin is present in the flavouring in an amount of less than 80% by weight of the flavouring. Preferably compositions of the invention comprise orange flavouring in an amount of 0.1 to 0.9 g per litre of solution to be made, such as 0.3 to 0.8 g per litre, for example 0.4 to 0.7 g per litre of solution to be made, for example 0.5 to 0.7 g per litre of solution to be made for example 0.6 g per litre. Preferably compositions of the invention comprise orange flavouring in an amount of more than 0.5g per litre, for example more than 0.55g per litre of solution to be made. The amount of flavouring needed to provide the most acceptable flavour depends to some degree on the levels of some of the other components present. If components (a) to (e) are present in amounts at the lower ends of their ranges, less flavouring is needed. This applies to some extent to the salts, but in particular to the organic acid component. It has been found that, for a composition comprising 8 to 12 g of organic acid component per litre of solution (for example 4.7g ascorbic acid and 5.9g sodium ascorbate), 0.2 to 0.65g of orange flavouring per litre of solution to be made (for example more than 0.5g, for example 0.6 g) provides a particularly acceptable solution. In a composition comprising less organic acid component, a lower amount of orange flavouring may be appropriate. For example, if 5 to 8g of organic acid component per litre of solution were present, then 0.2 to 0.5g of orange flavouring per litre of solution to be made might be adequate. If 12 to 15g of organic acid component per litre of solution were present, then 0.5 to 0.9g of orange flavouring per litre of solution to be made might be needed.

In the case where the flavouring comprises maltodextrin in an amount of 20 to 90% by weight of the flavouring, 0.3 to 0.8g of orange flavouring per litre of solution provides 0.06 to 0.72g maltodextrin per litre of solution, and thus maltodextrin makes up 0.042 to 0.73% of the dry composition by weight. In the case where the flavouring comprises maltodextrin in an amount of 35 to 80% by weight, 0.4 to 0.7g of orange flavouring per litre of solution provides 0.14 to 0.56g maltodextrin per litre of solution, and thus maltodextrin makes up 0.097 to 0.57% of the dry composition by weight. In the case where the flavouring comprises maltodextrin in an amount of 70 to 80% by weight of the flavouring, 0.4 to 0.7g of orange flavouring per litre of solution provides 0.28 to 0.56g maltodextrin per litre of solution and thus maltodextrin makes up 0.20 to 0.56% of the dry composition by weight. It has been found that a solution containing 0.442g maltodextrin per litre was preferred over solutions containing larger quantities of maltodextrin per litre. Accordingly, a preferred composition of the invention comprises less than 0.56g maltodextrin per litre of solution, for example it contains 0.28 to 0.56g, for example 0.35 to 0.5g, for example 0.41 to 0.47g maltodextrin per litre of solution to be made.

A preferred composition of the invention comprises 0.04 to 0.8% maltodextrin by weight of the dry composition. More preferably, it comprises 0.1 to 0.6% maltodextrin by weight of the dry composition, for example 0.2 to 0.6%.

Maltodextrin in a flavouring for use in a composition of the invention may be from any suitable source. For example, it may be maize maltodextrin (also known as corn maltodextrin), potato maltodextrin or wheat maltodextrin. For example, it is maize maltodextrin.

The orange flavouring may comprise a sugar, for example sucrose or dextrose (d-glucose), preferably dextrose. For example it may comprise dextrose in an amount of 10 to 30%, for example 15 to 25% by weight. An orange flavouring may comprise sucrose in an amount of from 30 to 50%, for example from 30 to 35% or from 35 to 45%. For example, an orange flavouring may comprise the same amount of sucrose as maltodextrin.

In the case where the flavouring comprises dextrose in an amount of 10 to 30%, by weight of the flavouring, 0.3 to 0.8g of orange flavouring per litre of solution provides 0.03 to 0.24g dextrose per litre of solution, and thus dextrose makes up 0.021 to 0.24 % of the dry composition by weight. In the case where the flavouring comprises dextrose in an amount of 15 to 25% by weight, 0.4 to 0.7g of orange flavouring per litre of solution provides 0.06 to 0.175g dextrose per litre of solution, and thus dextrose makes up 0.041 to 0.18 % of the dry composition by weight. It has been found that a solution containing 0.119g or 0.239g dextrose per litre was preferred over solutions containing less dextrose per litre. Accordingly, a preferred composition of the invention comprises more than 0.10g dextrose per litre, for example 0.10g to 0.25g dextrose per litre of solution to be made. In view of it being generally desirable to limit the amounts of sugars in bowel preparations, a preferred composition of the invention comprises less than 0.20g dextrose per litre of solution to be made, for example 0.100g to 0.180g, for example 0.110 to 0.130g dextrose per litre of solution to be made.

A preferred composition of the invention comprises 0.02 to 0.25% dextrose by weight of the dry composition. More preferably, it comprises 0.05 to 0.15% dextrose by weight of the dry composition, for example 0.075 to 0.125%.

Dextrose in a flavouring for use in a composition of the invention may be from any suitable source. For example, it may be derived from maize, rice, cassava, corn husk or sago. For example it is maize dextrose. In a composition of the invention that comprises maltodextrin and dextrose, both may be from the same source.

In the case where the flavouring comprises sucrose in an amount of 35 to 45% by weight of the flavouring, 0.4 to 0.7g of orange flavouring per litre of solution provides 0.14 to 0.315g sucrose per litre of solution and thus sucrose makes up 0.097 to 0.32 % of the dry composition by weight.

The orange flavouring may include nature-identical and/or natural flavouring preparations or components, for example orange oil, terpenes and terpenoids. Alternatively, the orange flavouring may be terpeneless, or may comprise terpenes at such a low level that they are regarded as terpeneless in the flavouring industry. In a preferred embodiment, the orange flavouring is substantially free from gum. Alternatively, it may comprise one or more gums, for example Gum Arabic, also known as acacia gum. The orange flavouring preferably comprises less than 2% by weight (relative to the weight of the flavouring) of organic components other than the flavouring preparations and components, for example less than 1%, for example less than 10ppm. Some components of flavourings can cause aqueous solutions of compositions comprising them to be cloudy. Some patients prefer drinking solutions that are clear.

Suitable flavourings are available from International Flavours and Fragrances Inc. (Duddery Hill, Haverhill, Suffolk, CB9 8LG, England), Ungerer & Company (Sealand Road, Chester, England CH1 4LP) or Firmenich (Firmenich UK Ltd., Hayes Road, Southall, Middlesex UB2 5NN).

The polyethylene glycol (PEG) used in compositions of the invention has an average molecular weight of 2500 to 4500 Daltons (Da). The PEG may have an average molecular weight of 3000 to 4000 Da. For example, the PEG may be PEG 3350 or PEG 4000 as defined in national pharmacopeias. Further examples of suitable PEGs recognized in some national pharmacopeias include Macrogols, for example Macrogol 4000. Optionally, the PEG used in compositions of the invention may comprise two or more different PEG compounds.

Compositions of the invention comprise PEG in an amount of 85 to 115 g per litre of solution to be made, preferably within a range wherein the lower limit is 90, 95 or 100 g per litre and the upper limit is, independently, 110 or 105 g per litre; for example 90 to 110 g per litre of solution to be made. For example a composition of the invention may comprise 100 g of PEG per litre, for example 100 g of PEG 3350 per litre of solution to be made.

Compositions of the invention comprise sodium sulphate in an amount of 6 to 9 g per litre of solution to be made, preferably within a range wherein the lower limit is 6.5, 7 or 7.5 g per litre and the upper limit is, independently, 8.5 or 8 g per litre; for example 6.5 to 8.5 g per litre of solution to be made. For example a composition of the invention may comprise 7.5 g of sodium sulphate per litre of solution to be made. The sodium sulphate in the compositions of the invention is preferably anhydrous.

Compositions of the invention comprise sodium chloride in an amount of 2 to 3g per litre of solution to be made, preferably within a range wherein the lower limit is 2.1, 2.2, 2.3, 2.4 or 2.5 g per litre and the upper limit is, independently, 2.9, 2.8, 2.7 or 2.6 g per litre; for example 2.5 to 2.9 g per litre of solution to be made. For example a composition of the invention may comprise 2.691 g of sodium chloride per litre of solution to be made.

Compositions of the invention comprise potassium chloride in an amount of 0.5 to 1.5 g per litre of solution to be made, preferably within a range wherein the lower limit is 0.6, 0.7, 0.8, 0.9 or 1.0 g per litre and the upper limit is, independently, 1.4, 1.3, 1.2 or 1.1 g per litre; for example 0.7 to 1.3 g per litre of solution to be made. For example a composition of the invention may comprise 1.105 g or 1.015 g of potassium chloride per litre of solution to be made.

Compositions of the invention are preferably substantially free from sodium bicarbonate.

Compositions of the invention comprise 5 to 15g of an organic acid component per litre of solution to be made. The organic acid component comprises an organic acid, one or more salts of an organic acid, or a mixture of an organic acid and one or more salts of an organic acid.

Preferably, the organic acid component is present in an amount within a range in which the lower limit is 6, 7, 8, 9 or 10 g per litre and the upper limit is, independently, 14, 13, 12 or 11 g per litre; for example 7 to 12 g per litre of solution to be made, such as 9 to 11 g per litre of solution to be made. For example, a composition of the invention may comprise 10.6 g of an organic acid component per litre.

The organic acid is preferably ascorbic and/or citric acid, for example ascorbic acid.

Preferred salts of an organic acid are alkali metal and alkaline earth metal salts, for example a salt may be selected from one or more of sodium, potassium, magnesium and calcium. For example, preferred salts of ascorbic acid include sodium ascorbate, potassium ascorbate, magnesium ascorbate and calcium ascorbate. A particularly preferred salt of ascorbic acid is sodium ascorbate. Preferred salts of citric acid include sodium citrate, potassium citrate, magnesium citrate and calcium citrate. A particularly preferred salt of citric acid is sodium citrate.

Preferably the organic acid component comprises both an organic acid and one or more salts of an organic acid. The organic acid salt or salts may be a salt(s) of the same acid as the organic acid(s) or it may be a salt or salts of a different organic acid(s). It is generally convenient for the organic acid salt to be a salt of the same acid as the organic acid. For example, a composition of the invention may comprise ascorbic acid and sodium ascorbate. A composition may comprise citric acid and sodium citrate. Alternatively, if the organic acid salt is a salt of a different organic acid, then a composition of the invention may, for example, comprise ascorbic acid and sodium citrate.

Preferably the organic acid and one or more salts of an organic acid are present in a weight ratio within the range of from 1:9 to 9:1. The organic acid and/or the one or more salts of an organic acid may, in practice, be provided as hydrates. If a hydrate is used, the weight and/or weight ratio mentioned here is calculated as the weight and/or weight ratio of organic acid or the one or more salts of an organic acid without water of hydration. Preferably the organic acid and one or more salts of an organic acid are present in a weight ratio within the range of from 2:8 to 8:2, more preferably 3:7 to 7:3, still more preferably 4:6 to 6:4. For example a composition of the invention may contain the organic acid and one or more salts of an organic acid in a weight ratio of 4.7 to 5.9, for example 4.7 g of ascorbic acid and 5.9 g of sodium ascorbate per litre of solution to be made.

In one embodiment, a composition of the invention is substantially free from an added component comprising citric acid or a salt thereof. Some orange flavourings may intrinsically comprise a small amount of citric acid, which is not considered substantial in this context.

Compositions of the invention may comprise one or more sweeteners. Sugar-based sweeteners are generally not suited for colon cleansing compositions because the delivery of unabsorbed sugars to the colon provides a substrate for bacteria. Such sugars may be metabolised by the bacteria to form explosive gases such as hydrogen and methane. The presence of explosive gases in the colon can be highly dangerous when electrical apparatus is to be used during colonoscopy or other procedures. Preferred sweeteners include aspartame, acesulfame potassium (acesulfame K), sucralose and saccharine, and/ or combinations thereof. For example, compositions of the invention may comprise one or both of aspartame and acesulfame potassium (acesulfame K). For example, compositions of the invention may comprise one or both of sucralose and acesulfame potassium (acesulfame K). Alternatively, compositions of the invention can be substantially free from added sweeteners, for example to minimize the number of different components in the compositions.

Aspartame may be present in an amount of 0.1 to 0.3 g per litre of solution to be made, more preferably in an amount within a range in which the lower limit is 0.12, 0.14, 0.16 or 0.18 g per litre and the upper limit is, independently, 0.24, 0.22 or 0.20 g per litre; for example 0.11 to 0.25. For example, a composition of the invention may comprise 0.10 to 0.13 (for example 0.117) or 0.16 to 0.19 (for example 0.175) or 0.22 to 0.25 (for example 0.233) g aspartame per litre. For example, a composition of the invention may comprise 0.175 g aspartame per litre of solution to be made. As compared with the lemonflavoured compositions of the prior art, it is possible to use a lower amount of aspartame than the prior art 0.233g per litre, when the solution includes an orange flavouring as in the current invention. For example, 0.175 g aspartame per litre of solution to be made is particularly preferred in a composition of the invention.

Acesulfame K may be present in an amount of 0.05 to 0.13 g per litre of solution to be made, more preferably within in a range in which the lower limit is 0.07 or 0.09 g per litre and the upper limit is, independently, 0.12 or 0.11 g per litre. For example, a composition of the invention may comprise 0.059 or 0.117 g acesulfame K per litre, for example 0.117 g acesulfame K per litre of solution to be made.

Whilst sweeteners can be used to give colon cleansing compositions a more acceptable taste, it is important that compositions are not unacceptably sweet. As mentioned above, creating a composition that is sufficiently acceptable to patients for them to drink around two litres is particularly challenging. It has been unexpectedly found that solutions of compositions of the invention comprising 0.6g of orange flavouring, 0.175g of aspartame and 0.117g of acesulfame K have an especially better taste than solutions of the prior art. These solutions are particularly acceptable for compositions that comprise maltodextrin (for example 0.28 to 0.56g per litre) and dextrose (for example 0.10 to 0.25g per litre).

In general it is not necessary for the compositions of the invention to include preservatives. Nevertheless, low levels of anti-oxidants or preservatives may be used if required.

Commercial compositions of the type described and claimed herein generally have manufacturing and regulatory tolerances of +/- 10%, or in some instances +/- 5%. Numerical values herein are accordingly preferably to be treated with the tolerances of +/- 10%, for example +/- 5%.

A preferred composition of the invention is a composition for admixture with water, wherein the composition is optionally presented in two or more parts and comprises, per litre of solution to be made, the following components:
(a) 90 to 110 g polyethylene glycol (PEG) with average molecular weight of 3000 to 4000 Da;
(b) 6.5 to 8.5 g sodium sulphate;
(c) 2.5 to 2.9 g sodium chloride;
(d) 0.7 to 1.3 g potassium chloride;
(e) 5 to 15 g of a mixture of an organic acid and one of more salts of an organic acid in a weight ratio of the organic acid to the one of more salts of an organic acid of 1:9 to 9:1; and
(f) 0.1 to 0.9g orange flavouring comprising maltodextrin in an amount of 70 to 90% by weight of the flavouring.

Another preferred composition, optionally in two or more parts, for admixture with water, comprises, per litre of solution to be made, the following components:
(a) 95 to 105 g polyethylene glycol (PEG) 3350 or 4000;
(b) 7 to 8 g sodium sulphate;
(c) 2.6 to 2.8 g sodium chloride;
(d) 0.8 to 1.2 g potassium chloride;
(e) 8 to 12 g of a mixture of an organic acid and one of more salts of an organic acid in a weight ratio of the organic acid to the one of more salts of an organic acid of 4:6 to 6:4; and
(f) 0.1 to 0.9g orange flavouring comprising maltodextrin in an amount of 70 to 90% by weight of the flavouring.

Compositions of the invention may comprise one of more sweeteners, although they may be substantially free from added sweeteners. A preferred composition comprises
(g) sweetener.

A particularly preferred composition, optionally in two or more parts, for admixture with water, comprises, per litre of solution to be made, the following components:
(a) 100 g polyethylene glycol (PEG) 3350;
(b) 7.5 g sodium sulphate;
(c) 2.691 g sodium chloride;
(d) 1.015 g potassium chloride;
(e1) 4.7 g ascorbic acid;
(e2) 5.9 g sodium ascorbate;
(f) 0.6 g orange flavouring.
(g1) 0.175 g aspartame; and
(g2) 0.117 g acesulfame K.

A composition may, for example, be in powder, granular or any other suitable physical form. For example it may be a dry powder composition. 'Dry' in this context means that the powder contains a sufficiently low level of moisture such that it is free flowing. The organic acid component may be coated. Such a coating helps to maintain stability of the organic acid component. Organic acids and salts of organic acids may otherwise be poorly stable in the presence of even small amounts of moisture.

The compositions of the invention may be provided in bulk form. The compositions may also be provided in unit dose form. A unit dose is generally an amount of composition suitable for preparing a defined volume of solution for administration to the patient in one treatment. The volume may be any suitable volume, for example, each unit dose may be suitable for making the total volume of solution of the composition of the invention required for use a single colon cleansing treatment. Alternatively, a unit dose may be suitable for making up to a defined volume, for example, a litre of solution of the composition of the invention, for use in one of the steps of a two step or multi-step cleansing regime.

The invention thus provides a unit dose composition for admixture with water, wherein the composition is optionally presented in two or more parts and comprises the following components:
(a) 85 to 115 g polyethylene glycol (PEG) having an average molecular weight of 2500 to 4500 Da;
(b) 6 to 9 g sodium sulphate;
(c) 2 to 3 g sodium chloride;
(d) 0.5 to 1.5 g potassium chloride;
(e) 5 to 15 g of an organic acid component; and
(f) orange flavouring comprising maltodextrin in an amount of 70 to 90% by weight of the flavouring.

Unit dose compositions of the invention have the preferred features described above in respect of the compositions of the invention.

As mentioned above, it has surprisingly been found that dry powder compositions exemplified herein remain free flowing and free from lumps in long term stability tests. Also disclosed is a dry powder composition comprising PEG and orange flavouring which composition (i) is substantially free from glucose, and (ii) is free-flowing and free from lumps after 3 months of storage at 25°C and 60% relative humidity. For example it is free-flowing and free from lumps after 6, 12, 18 or 24 months of storage under those conditions. The dry powder composition may comprise, in addition to the PEG and orange flavouring, an alkali earth metal or alkaline earth metal sulphate. An alkali metal or alkaline earth metal sulphate may, for example be selected from sodium sulphate, potassium sulphate and magnesium sulphate. Preferably sodium sulphate is present. For example, it may comprise more than one of sodium sulphate, potassium sulphate and magnesium sulphate, for example all three. Preferably the orange flavouring comprises dextrose or sucrose. Preferred orange flavourings are as described herein above. Preferably, the dry composition comprises aspartame. More preferably, the dry powder composition remains free-flowing after 6 months of storage at 25°C and 60% relative humidity. Preferably, the dry powder composition remains free-flowing after 3 months of storage at 40°C and 75% relative humidity; for example, the dry powder composition remains free-flowing after 6 months of storage at 40°C and 75% relative humidity.

The dry powder composition may comprise further electrolytes, for example selected from sodium chloride, potassium chloride and sodium bicarbonate. In one embodiment, the electrolytes may be sodium chloride, potassium chloride and sodium bicarbonate. In a further embodiment, the electrolytes may be sodium chloride, potassium chloride and sodium sulphate. In a further embodiment, the electrolytes may be sodium chloride, potassium chloride, sodium bicarbonate and sodium sulphate. The dry powder may further comprise one or more sweeteners, for example selected from acesulfame K, sucralose, aspartame and saccharine.

Also disclosed is a composition comprising an orange flavouring for admixture with water for the preparation of a bowel cleansing solution. Preferably, the orange flavouring is substantially free from glucose. Preferably the orange flavouring comprises dextrose and/or sucrose. Preferred orange flavourings are as described herein above. Preferably, the composition comprises aspartame. Preferably, the composition comprises acesulfame K. For example, the bowel cleansing solution may be a PEG-containing bowel cleansing solution comprising an orange flavouring comprising maltodextrin in an amount of 70 to 90% by weight of the flavouring. Such a bowel cleansing solution may further comprise electrolytes, for example selected from sodium chloride, potassium chloride, sodium bicarbonate and sodium sulphate. In a preferred embodiment, sodium suphate is present. In a preferred embodiment, the electrolytes are sodium chloride, potassium chloride and sodium sulphate. For example, sodium bicarbonate is not present. In an alternative embodiment, the electrolytes comprise sodium chloride, potassium chloride and sodium bicarbonate. In a further embodiment, the electrolytes are sodium chloride, potassium chloride, sodium bicarbonate and sodium sulphate. The solution may further comprise one or more sweeteners, for example selected from acesulfame K, sucralose, aspartame and saccharine. The solution may further comprise an organic acid component, for example an ascorbate component (for example a component comprising ascorbic acid and sodium ascorbate). Also disclosed is a bowel cleansing solution comprising an orange flavouring. Preferably, the orange flavouring is substantially free from glucose. Preferably the orange flavouring comprises dextrose and/or sucrose.

Preferred orange flavourings are as described herein above. Preferably, the solution comprises aspartame. Such a solution may have the characteristics described immediately above in relation to a composition for admixture with water. To be effective, a PEG-containing bowel cleansing solution is generally provided in a volume of over 500ml, for example from 500ml to 4000ml. For example, a PEG-containing colon cleansing solution may be provided in two doses to make up a treatment, each dose being of for example from 500ml to 2000ml, for example 1000ml. A composition for admixture with water for the preparation of a PEG-containing bowel cleansing solution provides a quantity of components for preparing such volumes of solutions.

As set out above, compositions of the invention are optionally in two or more parts. If the composition is in two or more parts, the organic acid component is preferably packaged separately from other components of the composition. For example, a first part of the composition may contain the polyethylene glycol, sodium sulphate, sodium chloride, potassium chloride, and orange flavouring components, and a second part may contain the organic acid component of the composition. In a further embodiment, the sodium sulphate may be packaged separately from other components. Such an embodiment may comprise two or more, for example three or more different parts. For example, a first part of the composition may contain polyethylene glycol, sodium chloride, potassium chloride, and orange flavouring components, a second part may contain the sodium sulphate, and a third part may contain the organic acid component of the composition. Each of the parts may comprise additional components or may comprise just the components stated. In some embodiments, the flavouring component may be provided in a separate part from the one or more other parts. In preferred embodiments however,.the flavouring component is packaged with the part containing the polyethylene glycol and/or sodium sulphate.

When the composition is provided in bulk form, for example, two or more parts may be measured or dispensed from metered dosing devices or other appropriate containers. When the composition is provided in unit dose form, two or more parts may be provided in sachets or other appropriate containers. The contents of the separate sachets or other appropriate containers are combined together at the point of use to form a complete composition of the invention.

It is convenient for the patient for a composition of the invention to be provided in the form of a kit, for example, a box, comprising the composition of the invention and instructions for its use. Such instructions may, for example, instruct use as described herein below. The kit preferably comprises the composition in unit dose form as described above. The kit may provide the components of a composition of the invention in any number of parts, for example two parts, three parts or four parts. For example a kit may comprise a first part of the composition provided in a first container containing polyethylene glycol, sodium sulphate, sodium chloride, potassium chloride and orange flavouring, and a second part of the composition provided in a second container containing the organic acid component, the contents of those containers together being for preparing a solution of a composition of the invention. For example, a container may be a sachet.

In an alternative embodiment of a kit, the sodium sulphate may be packaged separately from other components. Such an embodiment may comprise two or more, for example three or more, different parts and thus a kit may comprise a first part of the composition provided in a first container containing polyethylene glycol, sodium chloride, potassium chloride, and orange flavouring components, a second part of the composition provided in a container containing sodium sulphate, and a third part of the composition provided in a container containing the organic acid component of the composition. For example, a container may be a sachet.

In a further embodiment of a kit, the flavouring component may be provided in a separate container from the one or more other parts.

As discussed in further detail below, it may be desirable for a treatment to involve the patient taking two (or more) doses of compositions of the invention in separate steps of a treatment. In situations in which each unit dose of composition is intended for use in one of the steps of a two step or multi-step cleansing treatment, two or more first and second containers (for example sachets) may be provided in the kit, each first and second container (for example sachets) together being for preparing a solution of a composition of the invention, for example a litre of solution.

Accordingly, the present invention provides a kit comprising:
(a) a first container containing a first composition, said first composition comprising, per litre of solution to be made:
   (i) 85 to 115 g polyethylene glycol (PEG) having an average molecular weight of 2500 to 4500 Da;
   (ii) 6 to 9 g of sodium sulphate;
   (iii) 2 to 3 g of sodium chloride;
   (iv) 0.5 to 1.5 g potassium chloride; and
   (v) orange flavouring comprising maltodextrin in an amount of 70 to 90% by weight of the flavouring. and
(b) a second container containing a second composition comprising 5 to 15g of an organic acid component per litre of solution to be made.

A preferred kit in accordance with the invention comprises:
(a) a first container containing a first composition, said first composition comprising per litre of solution to be made:
   (i) 90 to 110 g polyethylene glycol (PEG) having an average molecular weight of 2500 to 4500 Da;
   (ii) 6.5 to 8.5 g of sodium sulphate;
   (iii) 0.7 to 1.3 g sodium chloride;
   (iv) 0.5 to 1.5 g potassium chloride; and
   (v) 0.1 to 0.9g orange flavouring comprising maltodextrin in an amount of 70 to 90% by weight of the flavouring; and
(b) a second container containing a second composition comprising 5 to 15 g of a mixture of an organic acid and one of more salts of an organic acid in a weight ratio of the organic acid to the one of more salts of an organic acid of 1:9 to 9:1 per litre of solution to be made.

Another preferred kit in accordance with the invention comprises:
(a) a first container containing a first composition, said first composition comprising per litre of solution to be made:
   (i) 95 to 105 g polyethylene glycol (PEG) 3350 or 4000;
   (ii) 7 to 8 g sodium sulphate;
   (iii) 2.5 to 2.8 g sodium chloride;
   (iv) 0.8 to 1.2 g potassium chloride; and
   (v) 0.4 to 0.7 g orange flavouring;
   and
(b) a second container containing a second composition comprising 8 to 12 g of a mixture of an organic acid and one of more salts of an organic acid in a weight ratio of the organic acid to the one of more salts of an organic acid of 4:6 to 6:4 per litre of solution to be made.

When the composition of the invention comprises one or more sweeteners, the sweetener(s) may be contained in the first container together with the polyethylene glycol, sodium sulphate, sodium chloride, potassium chloride, and orange flavouring.

A particularly preferred kit in accordance with the invention comprises:
(a) a first container containing a first composition, said first composition comprising per litre of solution to be made:
   (i) 100 g PEG 3350;
   (ii) 7.5 g sodium sulphate;
   (iii) 2.691 g sodium chloride;
   (iv) 1.015 g potassium chloride;
   (v) 0.6 g orange flavouring;
   (vi) 0.175 g aspartame; and
   (vii) 0.117 g acesulfame K;
   and
(b) a second container containing a second composition, said second composition comprising 4.7 g ascorbic acid and 5.9 g sodium ascorbate per litre of solution to be made.

Preferred containers include sachets, bottles, tubs, jugs or cans. For example, the composition may be provided in a container (for example a jug) of a set volume (for example 1 litre) such that the requisite amount of water can be readily measured out onto the powder. Sachets are also particularly preferred. It is preferred for one or both of the first and second compositions in a kit to be in dry powder form.

A composition in accordance with the invention may be provided as a composition, for example a dry composition, for making up into a solution as set out above, or alternatively as a solution in water. The invention provides a solution in water of the compositions described above. The present invention further provides an aqueous solution comprising water and:
(a) 85 to 115 g/l polyethylene glycol (PEG) having an average molecular weight of 2500 to 4500 Da;
(b) 6 to 9 g/l sodium sulphate;
(c) 2 to 3 g/l sodium chloride;
(d) 0.5 to 1.5 g/l potassium chloride;
(e) 5 to 15 g/l of an organic acid component; and
(f) orange flavouring comprising maltodextrin in an amount of 70 to 90% by weight of the flavouring.

Solutions according to the invention may be provided in any desired volume, for example from 0.2 to 2.5 litres, for example from 0.5 to 2 litres, for example 0.5 or 1 litre of solution in one or more containers. Solutions of the invention have the beneficial properties described above with reference to compositions. Solutions of the invention have the preferred features described above in respect of the compositions of the invention.A preferred solution of the invention comprises:
(a) 90 to 110 g/l polyethylene glycol (PEG) with average molecular weight of 3000 to 4000 Da;
(b) 6.5 to 8.5 g/l sodium sulphate;
(c) 2.5 to 2.9 g/l sodium chloride;
(d) 0.7 to 1.3 g/l potassium chloride;
(e) 5 to 15 g/l of a mixture of an organic acid and one of more salts of an organic acid in a weight ratio of the organic acid to the one of more salts of an organic acid of 1:9 to 9:1; and
(f) 0.4 to 0.7g/l orange flavouring.

Another preferred solution of the invention comprises:
(a) 95 to 105 g/l polyethylene glycol (PEG) 3350 or 4000;
(b) 7 to 8 g/l sodium sulphate;
(c) 2.6 to 2.8 g/l sodium chloride;
(d) 0.8 to 1.2 g/l potassium chloride;
(e) 8 to 12 g/l of a mixture of an organic acid and one of more salts of an organic acid in a weight ratio of the organic acid to the one of more salts of an organic acid of 4:6 to 6:4; and
(f) 0.4 to 0.7g/l orange flavouring.

A particularly preferred solution of the invention comprises:
(a) 100 g/l polyethylene glycol (PEG) 3350;
(b) 7.5 g/l sodium sulphate;
(c) 2.691 g/l sodium chloride;
(d) 1.015 g/l potassium chloride;
(el) 4.7 g/l ascorbic acid;
(e2) 5.9 g/l sodium ascorbate;
(f) 0.6 g/l orange flavouring;
(g1) 0.175 g/l aspartame; and
(g2) 0.117 g/l acesulfame K.

In the solutions of the invention described above, the quantities of the individual components listed do not include any solutes that may be present in the water used to prepare the solutions, for example, in hard water areas there may be significant amounts of Ca²⁺ and Mg²⁺ carbonates, bicarbonates or sulphates present in tap water.

The present invention also provides an aqueous solution comprising:
(a) 85 to 115 g/l polyethylene glycol (PEG) having an average molecular weight of 2500 to 4500 Da;
(b) 42 to 63 mmol/l sulphate ions; and
(c) 118 to 177 mmol/l sodium present as sodium ions;
(c1) 41 to 71 mmol/l chloride ions;
(d) 6.7 to 20 mmol/l potassium present as potassium ions;
(f) orange flavouring comprising maltodextrin in an amount of 70 to 90% by weight of the flavouring;
and additionally comprises:
(e) 5 to 15 g/l of an organic acid, one or more salts of an organic acid, or a mixture of an organic acid and one or more salts of an organic acid.

A preferred solution of the invention comprises:
(a) 90 to 110 g/l polyethylene glycol (PEG) with average molecular weight of 3000 to 4000 Da;
(b) 46 to 60 mmol/l sulphate ions; and
(c) 135 to 170 mmol/l sodium present as sodium ions;
(c1) 52 to 67 mmol/l chloride ions;
(d) 9.4 to 17 mmol/l potassium present as potassium ions;
(f) 0.4 to 0.7 g/l orange flavouring;
and additionally comprises:
(e) 5 to 15 g/l of a mixture of an organic acid and one or more salts of an organic acid in a weight ratio of the organic acid to the one of more salts of an organic acid of 1:9 to 9:1.

Another preferred solution of the invention comprises:
(a) 95 to 105 g/l polyethylene glycol (PEG) 3350 or 4000;
(b) 49 to 56 mmol/l sulphate ions; and
(c) 142 to 160 mmol/l sodium present as sodium ions;
(c1) 55 to 64 mmol/l chloride ions;
(d) 11 to 16 mmol/l potassium present as potassium ions;
(f) 0.4 to 0.7 g/l orange flavouring;
and additionally comprises:
(e) 8 to 12 g/l of a mixture of an organic acid and one or more salts of an organic acid in a weight ratio of the organic acid to the one of more salts of an organic acid of 4:6 to 6:4.

A particularly preferred solution of the invention comprises:
(a) 100 g/l polyethylene glycol (PEG) 3350;
(b) 52.8 mmol/l sulphate ions; and
(c) 152 mmol sodium present as sodium salts;
(c1) 59.7 mmol/l chloride ions;
(d) 13.6 mmol/l potassium present as potassium salts;
(f) 0.6 g/l orange flavouring;
(g1) 0.175 g/l aspartame;
and additionally comprises:
(e) 56.5 mmol/l ascorbate species, which ascorbate species comprise ascorbic acid, one or more salts of ascorbic acid, or a mixture of ascorbic acid and one or more salts of ascorbic acid; and
(g2) 0.58 mmol/l acesulfame species, which acesulfame species comprise acesulfame (6-methyl-1,2,3-oxathiazine-4(3*H*)-one 2,2-dioxide), one or more salts of acesulfame, or a mixture of acesulfame and one or more salts of acesulfame.

Another particularly preferred solution of the invention comprises:
(a) 100 g/l polyethylene glycol (PEG) 3350;
(b) 52.8 mmol/1 sulphate ions;
(c) 181 mmol/l sodium present as sodium salts;
(cl) 59.7 mmol/l chloride ions;
(d) 14.2 mmol/l potassium present as potassium salts;
(f) 0.6 g/l orange flavouring;
(g1) 0.175 g/l aspartame;
(e) 56.5 mmol/l ascorbate species, which ascorbate species comprise a mixture of ascorbic acid and ascorbate ions; and
(g2) 0.58 mmol/l acesulfame (6-methyl-1,2,3-oxathiazine-4(3*H*)-one 2,2-dioxide) ions.

The invention further provides a composition for admixture in water for the preparation of a solution of the invention.

The present invention further provides a composition for admixture with water, comprising the components polyethylene glycol (PEG) having an average molecular weight of 2500 to 4500 Da, sodium sulphate, sodium chloride, potassium chloride and an organic acid component, in the weight ratios:
(a) 85 to 115 polyethylene glycol (PEG) having an average molecular weight of 2500 to 4500 Da to
(b) 6 to 9 sodium sulphate to
(c) 2 to 3 sodium chloride to
(d) 0.5 to 1.5 potassium chloride to
(e) 5 to 15 of an organic acid component
and (f) orange flavouring comprising maltodextrin in an amount of 70 to 90% by weight of the flavouring.

Preferably, such a composition has the preferred features described above. Such a composition may comprise the various components in weight ratios based on absolute amounts, or amounts per litre, mentioned hereinabove. For example, such a composition comprises the following components in the following weight ratios:
(a) 100 polyethylene glycol (PEG) having an average molecular weight of 2500 to 4500 Da to
(b) 7.500 sodium sulphate to
(c) 2.691 sodium chloride to
(d) 1.015 potassium chloride to
(e1) 4.7 ascorbic acid to
(e2) 5.9 sodium ascorbate to
(f) 0.6 orange flavouring

Preferably, the orange flavouring comprises maltodextrin at a level of 70-80% of the flavouring by weight, for example providing maltodextrin at a weight ratio to the other components of 0.442. Preferably, the orange flavouring comprises dextrose, for example at a level of 15-25% of the flavouring by weight, for example providing dextrose at a weight ratio to the other components of 0.119. Preferably, the composition further comprises a sweetener, for example the following at the following weight ratios:
(g1) 0.175 aspartame, and
(g2) 0.117 acesulfame K.

Such a composition may be provided in two or more parts, for example with the components (e1) and (e2) packaged separately from the other components.

The compositions and solutions of the invention may be used to cleanse the colon prior to carrying out a diagnostic, therapeutic or surgical procedure on the colon, rectum or anus or elsewhere in the abdomen. The diagnostic or surgical procedure may, for example, be colonoscopy, barium enema examination, sigmoidoscopy or colon surgery.

The composition, solution, kit or unit dose of the invention may be provided for use as a medicament, for example for use in cleansing the colon.

Accordingly, the present invention also provides a method of cleansing the colon of a subject in which the subject ingests an oral colon cleansing solution comprising:
(a) 85 to 115 g/l polyethylene glycol (PEG) having an average molecular weight of 2500 to 4500 Da;
(b) 6 to 9 g/l of sodium sulphate;
(c) 2 to 3 g/l sodium chloride;
(d) 0.5 to 1.5 g/l potassium chloride;
(e) 5 to 15 g/l of an organic acid component; and
(f) orange flavouring comprising maltodextrin in an amount of 70 to 90% by weight of the flavouring.

A solution for use in a method of the invention has the preferred features described above in respect of the compositions and/or solutions of the invention.

The exact quantity of solution to be administered in a method of the invention will depend on the subject being treated and the subject's particular situation. For example, the subject may be an adult human. An appropriate volume of cleansing solution for an adult human may be from 1.5 to 4 litres, while a proportionately smaller volume of cleansing solution is appropriate in the treatment of small children and a higher volume of cleansing solution is appropriate in patients with prolonged colonic transit times. Typically, 2 litres of the cleansing solution is administered to an adult human. For example, 1 to 2 litres of the colon cleansing solution is ingested by the subject. In one embodiment, the subject additionally ingests a quantity of clear liquid, for example one litre.

Preferably, the total volume of solution is administered over 1 to 4 hours. The 1 to 4 hours may be in a continuous period or a discontinuous period. In an administration regime using a discontinuous period, a portion of the solution, typically approximately half, may be administered the evening before the diagnostic, therapeutic or surgical procedure is to be carried out, with the remainder of the solution being administered on the day of, and before, the procedure.

Discontinuous administration may also involve administering first a portion of the cleansing solution, followed by administration of a clear fluid.

In a preferred regime for taking a cleansing solution of the invention, a 2 litre treatment is taken by the subject. The treatment may be taken as a divided treatment with 1 litre of cleansing solution being taken in the evening before a clinical examination or procedure and I litre being taken in the early morning of the day of the examination or procedure. Alternatively, 2 litres of the cleansing solution may be taken in the evening before the examination or procedure. Subjects are told not to take any solid food from when they start to take the cleansing solution until after the examination or procedure.

Subjects may be told to prepare a first litre of the cleansing solution by dissolving the provided dry powder (for example provided in two sachets with the organic acid component in one sachet and the remaining components in a separate sachet) in water, and then to take the solution over one to two hours, for example by drinking a glassful every 10-15 minutes. They may be told to then make up and drink the second litre of cleansing solution, and to drink that. Subjects may be recommended to drink a further one litre of clear liquid to prevent them from feeling very thirsty and becoming dehydrated. Water, clear soup, fruit juice (without pulp), soft drinks, tea or coffee (without milk) are all suitable.

A possible variant on the above regime is a regime in which the full treatment dose for colon cleansing for adult subjects is 2 litres (approximately 64 fluid ounces) of cleansing solution (with 1 additional litre of clear fluids) taken orally prior to the colonoscopy or other examination or procedure in one of the following ways:
1) Split-dose regimen: The evening before the colonoscopy or other examination or procedure, the first litre (approximately 32 fluid ounces) of cleansing solution is taken over one hour (one 250ml / 8 fluid ounce glass every 15 minutes) and then 0.5 litres (approximately 16 fluid ounces) of clear fluid are drunk. Then, on the morning of the colonoscopy or other examination or procedure, the second litre (approximately 32 fluid ounces) of cleansing solution is taken over one hour and then 0.5 litres (approximately 16 fluid ounces) of clear liquid is drunk at least one hour prior to the start of the colonoscopy.
**2) Evening-only (full-dose) regimen:** Around 6 pm in the evening before the colonoscopy or other examination or procedure, a first litre of cleansing solution is taken over one hour (one 250ml / 8 fluid ounce glass every 15 minutes) and then about 1.5 hours later a second litre of cleansing solution (approximately 32 fluid ounces) is taken over one hour. In addition, 1 litre (approximately 32 fluid ounces) of additional clear liquid is taken during the evening before the colonoscopy or other examination or procedure.

### Examples

The following non-limiting Examples illustrate the invention.

The flavourings tested included Orange Flavour 1, Orange Flavour 2 and Orange Flavour 3. MOVIPREP® (as available from Norgine Limited, New Road, Hengoed, Mid Glamorgan, CF82 8SJ, UK), a currently marketed colon cleansing preparation comprising a lemon flavour (Ungerer lemon V3938-1N1), was used as a comparison.

Orange Flavour 1 comprises 72-76% by weight maize maltodextrin and 19-21% by weight maize dextrose, nature identical flavouring substances, natural flavour preparations and natural flavouring substances. It is substantially free from sucrose.

Orange Flavour 2 comprises 35-50% by weight maize maltodextrin and 30-35% by weight sucrose. It also comprises flavouring preparations, starch and gum. It is substantially free from dextrose.

Orange Flavour 3 comprises 90-95% by weight maltodextrin, terpeneless orange oil and gum. It is substantially free from dextrose and substantially free from sucrose.
(% by weight being relative to the weight of the flavour.)

In each of Examples 1 to 4, the ascorbic acid and sodium ascorbate were provided in one sachet (sachet B) as shown in Table 1b, and the other components were provided in a separate sachet (sachet A) as shown in Table 1a. Each Sachet A used in Examples 1, 2 and 4 also comprised the flavouring and sweetener in the respective amounts set out in Tables 2, 3 and 5 respectively. In each case, the sachets A and B were mixed together and made up to one litre with water (to make a "sample").

**Table 1a: Composition per litre of sample - Sachet A**

| **Component** | **Quantity (g)** |
|---|---|
| PEG 3350 | 100.000 |
| Sodium Sulphate | 7.500 |
| Sodium Chloride | 2.691 |
| Potassium Chloride | 1.015 |
| Flavour/Sweetener | See Tables 2-5 |

**Table 1b: Composition per litre of sample - Sachet B**

| | |
|---|---|
| Ascorbic Acid | 4.700 |
| Sodium Ascorbate | 5.900 |

### Example 1: Comparison of palatability of different flavoured colon cleansing preparations

12 healthy volunteers in a Western country were given samples of 5 differently flavoured colon cleansing preparations and asked to evaluate the palatability of each on a scale from 1 (low) to 10 (high).

The results of the palatability tests on chilled solutions are shown in Table 2.

**Table 2: Palatability scores**

| **Ex No** | **Flavour** | **Flavour (g)** | **Aspartame (g)** | **Acesulfame K (g)** | **Taste Score (average)** |
|---|---|---|---|---|---|
| 1-1 | Orange Flavour 1 | 0.4 | 0.175 | 0.117 | **6.17** |
| 1-2 | Orange Flavour 1 | 0.5 | 0.175 | 0.117 | **6.08** |
| 1-3 | Orange Flavour 2 | 0.6 | 0.175 | 0.117 | **5.92** |
| 1-4 | Orange Flavour 3 | 0.4 | 0.175 | 0.117 | **4.92** |
| Comparative 1-5 | MOVIPREP (Ungerer Lemon V3938-1N1) | 0.34 | 0.233 | 0.117 | **4.92** |

As is seen in Table 2, the solutions comprising Orange Flavour 1 or Orange Flavour 2 were preferred over the lemon flavoured solution of the prior art. Orange Flavour 3 was not inferior to the prior art solution. The preferred solutions 1-1, 1-2 and 1-3 contained Orange Flavour 1 and Orange Flavour 2, which comprised maltodextrin at the levels mentioned above. The solution comprising Orange Flavour 1 was especially preferred. The flavouring in that solution includes dextrose. Solutions 1-1, 1-2 were clear; solution 1-4 was slightly opaque, and solution 1-3 was cloudy.

### Example 2: Comparison of palatability of different orange flavoured colon cleansing solutions

A group of healthy volunteers in an Eastern country were given samples of 4 differently flavoured colon cleansing solutions and asked to evaluate the palatability of each of the solutions on a scale from 1 (low) to 10 (high). The results of the palatability tests are shown in Table 3. The number of subjects given each solution is shown in the table.

**Table 3: Palatability scores**

| **Ex No.** | **Flavour** | **n** | **Flavour (g)** | **Aspartame (g)** | **Acesulfame Potassium (g)** | **Score (average)** |
|---|---|---|---|---|---|---|
| 2-1 | Orange Flavour 1 | 10 | 0.225 | 0.117 | 0.059 | **6.6** |
| 2-2 | Orange Flavour 1 | 7 | 0.6 | 0.175 | 0.117 | **6.86** |
| 2-3 | Orange Flavour 2 | 9 | 0.6 | 0.175 | 0.117 | **5.8** |
| 2-4 | Orange Flavour 3 | 5 | 0.6 | 0.175 | 0.117 | **4.8** |

As is seen in Table 3, the Orange flavoured solutions in which the flavouring includes maltodextrin at a level as in Orange Flavour 1 and Orange Flavour 2 were preferred. The solution comprising Orange Flavour 1 was especially preferred, particularly when that flavouring was present at a level of 0.6g per litre of solution. The flavouring in that solution also includes dextrose.

### Example 3: Comparison of palatability of different flavoured colon cleansing solutions

An additional study was carried out in which 15 healthy volunteers in a Western country were given samples of 4 differently flavoured colon cleansing solutions (comprising Orange flavour 1, Orange flavour 2, Orange flavour 3 or prior art Lemon) and asked to evaluate the palatability of each of the chilled solutions on a scale from 1 (low) to 10 (high). Taste perceptions can vary between cultures, but it is more efficient to manufacture and register the same pharmaceutical product for several countries. Accordingly, the results of the taste tests in Example 1 and Example 2 and the additional study were combined to give the average palatability as shown in Table 4.

**Table 4: Average Palatability scores**

| **Flavour** | **Total n** | **Flavour (g)** | **Aspartame (g)** | **Acesulfame Potassium (g)** | **Score (average)** |
|---|---|---|---|---|---|
| Orange Flavour | 124 | 0.6, 0.5 or 0.4 | 0.175 or 0.117 | 0.117 or 0.059 | **5.62** |
| MOVIPREP (Ungerer Lemon V3938-1N1) | 27 | 0.34 | 0.233 | 0.117 | **4.63** |

As is seen in Table 4, the Orange flavoured solutions were preferred over the lemon flavoured solution of the prior art.

### Example 4: Comparison of palatability of different levels of sweetener and Orange flavour

4 healthy volunteers were given samples of 8 different colon cleansing solutions, each comprising a different amount of sweetener (either aspartame or sucralose) and flavour (Orange Flavour 1), and asked to evaluate the palatability of each of the solutions on a scale from 1 (low) to 10 (high). In addition to the flavour and sweetener mentioned in Table 5, the samples also comprised 0.117g acesulfame K. The responses are summarised in Table 5. For three of the samples, only three of the volunteers provided an evaluation regarding the flavour level.

**Table 5: Palatability scores**

| **Ex No** | **Amount of Flavour 1 (g) and Sweetener (g)** | **Average Rating** | **Sweetness Level** | | | **Perceived Flavour Level** | | |
|---|---|---|---|---|---|---|---|---|
| | | | **Too High** | **OK** | **Too Low** | **Too High** | **OK** | **Too Low** |
| 4-1 | Orange Fl 1 0.6 | **7.50** | 2 | 2 | - | - | 3 | 1 |
| | Aspartame 0.233 | | | | | | | |
| 4-2 | Orange Fl 1 0.6 | **8.25** | - | 4 | - | - | 4 | |
| | Aspartame 0.175 | | | | | | | |
| 4-3 | Orange Fl 1 0.5 | **7.00** | 1 | 2 | 1 | - | 2 | 2 |
| | Aspartame 0.233 | | | | | | | |
| 4-4 | Orange Fl 1 0.5 | **7.00** | - | 4 | - | - | 2 | 2 |
| | Aspartame 0.175 | | | | | | | |
| 4-5 | Orange Fl 1 0.6 | **6.25** | - | 3 | 1 | - | 1* | 2* |
| | Sucralose 0.233 | | | | | | | |
| 4-6 | Orange Fl 1 0.6 | **5.75** | - | 2 | 2 | - | - | 3* |
| | Sucralose 0.175 | | | | | | | |
| 4-7 | Orange Fl 1 0.5 | **7.25** | - | 3 | 1 | - | 3* | - |
| | Sucralose 0.233 | | | | | | | |
| 4-8 | Orange Fl 1 0.5 | **5.5** | - | 2 | 2 | - | 1 | 3 |
| | Sucralose 0.175 | | | | | | | |

| | | | | | | | | |
|---|---|---|---|---|---|---|---|---|
| * results were only obtained for 3 subjects for these data points. | | | | | | | | |

As is seen in Table 5, Orange Flavour 1 at a concentration of 0.6g per litre with aspartame at a concentration of 0.175g per litre was found to be the most palatable combination. That is the combination in Example 4-2, which is the same as Example 2-2.

### Example 5: Comparison of palatability of orange-flavoured colon cleansing preparations

7 healthy volunteers were given samples of 6 different orange-flavoured colon cleansing solutions asked to evaluate the palatability of each on a scale from 1 (low) to 10 (high). One of the solutions (5-3) comprised Orange flavour 1 as used above in Examples 1 to 4, and the other five solutions comprised different variations of that flavour. The Sachet A composition contained the components of Table 1a together with 0.175g per litre aspartame and 0.117g per litre Acesulfame K; and orange flavouring. The Sachet B composition was as shown in Table 1b. The 6 different orange flavourings each comprised the same amounts of nature identical flavouring substances, natural flavour preparations and natural flavouring substances, and they were substantially free from sucrose. However, each comprised different proportions of maize maltodextrin and maize dextrose, as shown in Table 6a, with the amounts presented as % figures in Table 6b. Example 5-3 is identical to Examples 2-2 and 4-2. The results of the palatability tests are shown in Table 6a.

**Table 6a: Palatability scores**

| **Ex No** | **Weight of maltodextrin (g)** | **Weight of dextrose (g)** | **Weight of other flavouring components (g)** | **Taste Score (average)** |
|---|---|---|---|---|
| 5-1 | 0.442 | 0.000 | 0.039 | **4.3** |
| 5-2 | 0.442 | 0.060 | 0.039 | **4.1** |
| 5-3 | 0.442 | 0.119 | 0.039 | **4.7** |
| 5-4 | 0.442 | 0.239 | 0.039 | **5.4** |
| 5-5 | 0.663 | 0.119 | 0.039 | **4.3** |
| 5-6 | 0.884 | 0.119 | 0.039 | **4.6** |

**Table 6b: Amounts of components as percentages by weight**

| **Ex No** | **Maltodextrin** | | | **Dextrose** | | |
|---|---|---|---|---|---|---|
| | Weight (g) | % by weight of flavour | % by weight of dry composition | Weight (g) | % by weight of flavour | % by weight of dry composition |
| 5-1 | 0.442 | 91.89% | 0.36% | 0.000 | 0% | 0% |
| 5-2 | 0.442 | 81.70% | 0.36% | 0.060 | 11.09% | 0.05% |
| 5-3 | 0.442 | 73.67% | 0.36% | 0.119 | 19.83% | 0.10% |
| 5-4 | 0.442 | 61.39% | 0.36% | 0.239 | 33.19% | 0.19% |
| 5-5 | 0.663 | 80.76% | 0.54% | 0.119 | 14.50% | 0.10% |
| 5-6 | 0.884 | 84.84% | 0.72% | 0.119 | 11.42% | 0.10% |

As is seen in Table 6a by comparison of the taste scores for Examples 5-1 to 5-4 (all of which comprise the same amount of maltodextrin), the examples containing 0.119g or 0.239g dextrose were preferred over the samples containing less dextrose. A comparison of the taste scores for Examples 5-3, 5-5 and 5-6 (all of which comprise the same amount of dextrose) shows that Example 5-3 comprising 0.442g maltodextrin was preferred over Examples 5-5 and 5-6 comprising larger amounts of maltodextrin.

It is seen that samples comprising orange flavour containing 0.119g or 0.239g dextrose per litre are preferred over samples containing less dextrose, and that samples comprising orange flavour containing 0.442g maltodextrin per litre are preferred over samples containing larger amounts of maltodextrin.

### Example 6a: Long term stability of a composition of the invention

Samples containing the components of Table 1a with 0.600 g of Orange Flavour 1, or 0.600 g of Orange Flavour 3 were tested for long term stability.

**Table 7: Composition of dry powders for stability testing:**

| **Component** | **Example 6-1** | **Example 6-2** |
|---|---|---|
| | **Quantity (g)** | **Quantity (g)** |
| PEG 3350 | 100.000 | 100.000 |
| Sodium Sulphate (Anhydrous) | 7.500 | 7.500 |
| Sodium Chloride | 2.691 | 2.691 |
| Potassium Chloride | 1.015 | 1.015 |
| Aspartame | 0.175 | 0.175 |
| Acesulfame Potassium | 0.117 | 0.117 |
| Orange Flavour 1 | 0.600 | - |
| Orange Flavour 3 | - | 0.600 |

The compositions of Example 6-1 thus comprised the same components in the same amounts as Examples 2-2, 4-2 and 5-3. The compositions of Example 6-2 comprised the same components in the same amounts as Example 2-4. The components shown in Table 7 were packaged into sachets (of the same type as used for the marketed product sold under the name MOVIPREP®) and sealed. Three different manufactured batches of the composition of Example 6-1 were tested. Two different manufactured batches of the composition of Example 6-2 were tested. Sachets were placed under different conditions:
a) 25 °C / 60% Relative Humidity to 24 months for Example 6-1 and to 12 months for Example 6-2
b) 40 °C / 75% Relative Humidity to 6 months for Example 6-1 and 6-2 The batches of Example 6-1 under conditions a) were assessed after 1, 3, 6, 9, 12, 18 and 24 months. The batches of Example 6-2 under conditions a) were assessed after 1, 3, 6, 9 and 12 months. The batches of both Examples under conditions b) were assessed after 1, 3 and 6 months. The assessment was under the following criteria:

| Description of visual appearance | Opalescence |
|---|---|
| Colour of solution | PEG3350 content |
| Potassium content | Sodium content |
| Chloride content | Sulphate content |
| Moisture content | pH |
| Reconstituion time | Formaldehyde content |
| Taste | Odour |

All of the samples were within the acceptable tolerances for each criterion under both sets of conditions (25°C/60%RH and 40°C/75%RH). In particular, all of the samples were free flowing with no visible lumps at each of the time points and under both sets of conditions.

### Comparative Example 6b: Comparison with long term stability of a prior art composition

Laxido Orange is a constipation treatment marketed by Galen Limited (see http://www.galen.co.uk) that comprises 13.125g PEG 3350, 350.7mg sodium chloride, 178.5mg sodium hydrogen carbonate, 46.6mg potassium chloride, acesulfame potassium and orange flavouring, and is supplied as a dry powder.

Samples of Laxido Orange dry powder were subjected to 6 month stability tests as described in Example 6a. The samples were within the acceptable tolerances for most of the criteria under both sets of conditions (25°C/60%RH and 40°C/75%RH). However, the samples had visible small lumps at both the 3 month and the 6 month time points, under both sets of conditions.

Samples of Laxido Orange thus deteriorated over the 3 or 6 month testing period. As seen above in Example 6a, the orange flavoured compositions of the invention did not form lumps in the 3 or 6 month stability tests.

## Claims

1. A dry composition for admixture with water, **characterized in that** the composition is optionally presented in two or more parts and comprises, per litre of solution to be made, the following components:
(a) 85 to 115 g polyethylene glycol (PEG) having an average molecular weight of 2500 to 4500;
(b) 6 to 9 g sodium sulphate;
(c) 2 to 3 g sodium chloride;
(d) 0.5 to 1.5 g potassium chloride;
(e) 5 to 15 g of an organic acid component; and
(f) orange flavouring comprising maltodextrin in an amount of 70 to 90% by weight of the flavouring.

2. A composition as claimed in claim 1 wherein the orange flavouring comprises maltodextrin in an amount of 70 to 80% by weight of the flavouring.

3. A composition as claimed in claim 2 wherein the maltodextrin is maize maltodextrin.

4. A composition as claimed in any one of claims 1 to 3 wherein the orange flavouring comprises dextrose in an amount of 15 to 25% by weight of the flavouring.

5. A composition as claimed in any one of claims 1 to 4 wherein the orange flavouring is free from gum.

6. A composition as claimed in any one of claims 1 to 5 wherein the orange flavouring comprises less than 2% by weight of organic components other than flavouring preparations and components.

7. A composition as claimed in any one of claims 1 to 6 wherein the orange flavouring is present in a quantity of 0.1 to 0.9g per litre of solution to be made.

8. A composition as claimed in any one of claims 1 to 7 wherein the composition comprises a sweetener, for example one or both of aspartame and acesulfame potassium (acesulfame K).

9. A composition as claimed in claim 8 wherein aspartame is present in a quantity of 0.175g per litre of solution to be made.

10. A composition as claimed in claim 8 or claim 9,wherein acesulfame potassium is present in a quantity of 0.117g per litre of solution to be made.

11. A composition as claimed in any one of claims 1 to 10 comprising ascorbic acid and sodium ascorbate.

12. A composition as claimed in any one of claims 1 to 11 which is free from sodium bicarbonate.

13. A composition as claimed in any one of claims 1 to 12 comprising, per litre of solution to be made, the following components:
(a) 100 g polyethylene glycol (PEG) 3350;
(b) 7.5 g sodium sulphate;
(c) 2.691 g sodium chloride;
(d) 1.015 g potassium chloride;
(e1) 4.7 g ascorbic acid;
(e2) 5.9 g sodium ascorbate;
(f) 0.6 g orange flavouring.
(g1) 0.175 g aspartame; and
(g2) 0.117 g acesulfame K.

14. An aqueous solution comprising water and:
(a) 85 to 115 g/l polyethylene glycol (PEG) having an average molecular weight of 2500 to 4500;
(b) 6 to 9 g/l sodium sulphate;
(c) 2 to 3 g/l sodium chloride;
(d) 0.5 to 1.5 g/l potassium chloride;
(e) 5 to 15 g/l of an organic acid component; and
(f) orange flavouring comprising maltodextrin in an amount of 70 to 90% by weight of the flavouring.

15. A kit that provides the components of a composition as claimed in any one of claims 1 to 13 in two or more parts.

16. A kit as claimed in claim 15 comprising:
(a) a first container containing a first composition, said first composition comprising per litre of solution to be made:
(i) 85 to 115 g polyethylene glycol (PEG) having an average molecular weight of 2500 to 4500;
(ii) 6 to 9 g of sodium sulphate;
(iii) 2 to 3 g of sodium chloride;
(iv) 0.5 to 1.5 g potassium chloride; and
(v) orange flavouring comprising maltodextrin in an amount of 70 to 90% by weight of the flavouring;
and
(b) a second container containing a second composition comprising 5 to 15g of an organic acid component per litre of solution to be made.

17. A kit as claimed in claim 16 comprising two sachets having the components:
SACHET 1
| | |
|---|---|
| PEG 3350: | 100.000 g |
| Sodium Sulphate: | 7.500 g |
| Sodium Chloride: | 2.691 g |
| Potassium Chloride: | 1.015 g |
| Aspartame: | 0.175 g |
| Acesulfame K: | 0.117 g |
| Orange flavouring: | 0.6 g |
SACHET 2
| | |
|---|---|
| Ascorbic Acid: | 4.700 g |
| Sodium Ascorbate: | 5.900 g |

18. A unit dose composition for admixture with water wherein the composition is optionally presented in two or more parts and comprises the following components:
(a) 85 to 115 g polyethylene glycol (PEG) having an average molecular weight of 2500 to 4500;
(b) 6 to 9 g sodium sulphate;
(c) 2 to 3 g sodium chloride;
(d) 0.5 to 1.5 g potassium chloride;
(e) 5 to 15 g of an organic acid component; and
(f) orange flavouring comprising maltodextrin in an amount of 70 to 90% by weight of the flavouring.

19. A solution as claimed in claim 14, a kit as claimed in claim 15, 16 or 17, or a unit dose as claimed in claim 18 **characterized in that** the solution, kit or unit dose has one or more of the features of a composition recited in any one or more of claims 1 to 13.

20. An oral colon cleansing solution comprising:
(a) 85 to 115 g/l polyethylene glycol (PEG) having an average molecular weight of 2500 to 4500;
(b) 6 to 9 g/l of sodium sulphate;
(c) 2 to 3 g/l sodium chloride;
(d) 0.5 to 1.5 g/l potassium chloride;
(e) 5 to 15 g/l of an organic acid component; and
(f) orange flavouring comprising maltodextrin in an amount of 70 to 90% by weight of the flavouring.
for use in a method of cleaning the colon of a subject, in which the subject ingests the solution.

21. An oral colon cleansing solution as claimed in claim 20 in which 1 to 2 litres of the colon cleansing solution is ingested by the subject.

22. An oral colon cleansing solution as claimed in claim 21 in which the colon cleansing solution is taken as a divided treatment with 1 litre of cleansing solution being taken in the evening before a clinical examination or procedure and 1 litre being taken in the early morning of the day of an examination or procedure.

23. An oral colon cleansing solution as claimed in claim 20 or 21 in which the colon cleansing solution is taken in the evening before an examination or procedure.

24. An oral colon cleansing solution as claimed in any one of claims 20 to 23 in which the subject additionally ingests a further one litre of clear liquid.

25. An oral colon cleansing solution as claimed in claim 20 in which the evening before a colonoscopy or other examination or procedure, a first litre of cleansing solution is ingested over approximately one hour and then 0.5 litres of clear fluid are ingested, and, on the morning of the colonoscopy or other examination or procedure, a second litre of cleansing solution is ingested over approximately one hour followed by 0.5 litres of clear liquid.

26. An oral colon cleansing solution claimed in claim 20 in which the evening before a colonoscopy or other examination or procedure, a first litre of cleansing solution is ingested over approximately one hour and then about 1.5 hours later a second litre of cleansing solution is ingested over approximately one hour, with an additional 1 litre of additional clear liquid being ingested during the evening.

27. A composition as claimed in any one of claims 1 to 13, or a solution as claimed in claim 14 or 19, for use as a medicament.

## Patentansprüche

1. Trockene Zusammensetzung zum Mischen mit Wasser, **dadurch gekennzeichnet, dass** die Zusammensetzung gegebenenfalls in zwei oder mehr Teilen vorliegt und pro Liter herzustellender Lösung die folgenden Komponenten umfasst:
(a) 85 bis 115 g Polyethylenglykol (PEG) mit einem durchschnittlichen Molekulargewicht von 2500 bis 4500;
(b) 6 bis 9 g Natriumsulfat;
(c) 2 bis 3 g Natriumchlorid;
(d) 0,5 bis 1,5 g Kaliumchlorid;
(e) 5 bis 15 g einer organischen Säurekomponente; und
(f) Orangenaroma, umfassend Maltodextrin in einer Menge von 70 bis 90 Gew.-% des Aromas.

2. Zusammensetzung nach Anspruch 1, wobei das Orangenaroma Maltodextrin in einer Menge von 70 bis 80 Gew.-% des Aromas umfasst.

3. Zusammensetzung nach Anspruch 2, wobei das Maltodextrin Mais-Maltodextrin ist.

4. Zusammensetzung nach einem der Ansprüche 1 bis 3, wobei das Orangenaroma Dextrose in einer Menge von 15 bis 25 Gew.-% des Aromas umfasst.

5. Zusammensetzung nach einem der Ansprüche 1 bis 4, wobei das Orangenaroma frei von Gummi ist.

6. Zusammensetzung nach einem der Ansprüche 1 bis 5, wobei das Orangenaroma weniger als 2 Gew.-% andere organische Komponenten als Aromapräparate und -komponenten umfasst.

7. Zusammensetzung nach einem der Ansprüche 1 bis 6, wobei das Orangenaroma in einer Menge von 0,1 bis 0,9 g pro Liter herzustellender Lösung vorliegt.

8. Zusammensetzung nach einem der Ansprüche 1 bis 7, wobei die Zusammensetzung einen Süßstoff umfasst, beispielsweise einen oder beide von Aspartam und Acesulfam-Kalium (Acesulfam K) .

9. Zusammensetzung nach Anspruch 8, wobei Aspartam in einer Menge von 0,175 g pro Liter herzustellender Lösung vorliegt.

10. Zusammensetzung nach Anspruch 8 oder Anspruch 9, wobei Acesulfam-Kalium in einer Menge von 0,117 g pro Liter herzustellender Lösung vorliegt.

11. Zusammensetzung nach einem der Ansprüche 1 bis 10, die Ascorbinsäure und Natriumascorbat umfasst.

12. Zusammensetzung nach einem der Ansprüche 1 bis 11, die frei von Natriumbicarbonat ist.

13. Zusammensetzung nach einem der Ansprüche 1 bis 12, die pro Liter herzustellender Lösung die folgenden Komponenten umfasst:
(a) 100 g Polyethylenglykol (PEG) 3350;
(b) 7,5 g Natriumsulfat;
(c) 2,691 g Natriumchlorid;
(d) 1,015 g Kaliumchlorid;
(el) 4,7 g Ascorbinsäure;
(e2) 5,9 g Natriumascorbat;
(f) 0,6 g Orangenaroma;
(g1) 0,175 g Aspartam; und
(g2) 0,117 g Acesulfam K.

14. Wässrige Lösung, umfassend Wasser und:
(a) 85 bis 115 g/l Polyethylenglykol (PEG) mit einem durchschnittlichen Molekulargewicht von 2500 bis 4500;
(b) 6 bis 9 g/l Natriumsulfat;
(c) 2 bis 3 g/l Natriumchlorid;
(d) 0,5 bis 1,5 g/l Kaliumchlorid;
(e) 5 bis 15 g/l einer organischen Säurekomponente; und
(f) Orangenaroma, umfassend Maltodextrin in einer Menge von 70 bis 90 Gew.-% des Aromas.

15. Kit, das die Komponenten einer Zusammensetzung gemäß einem der Ansprüche 1 bis 13 in zwei oder mehr Teilen bereitstellt.

16. Kit nach Anspruch 15, umfassend:
(a) einen ersten Behälter, der eine erste Zusammensetzung enthält, wobei die erste Zusammensetzung pro Liter herzustellender Lösung umfasst:
(i) 85 bis 115 g Polyethylenglykol (PEG) mit einem durchschnittlichen Molekulargewicht von 2500 bis 4500;
(ii) 6 bis 9 g Natriumsulfat;
(iii) 2 bis 3 g Natriumchlorid;
(iv) 0,5 bis 1,5 g Kaliumchlorid; und
(v) Orangenaroma, umfassend Maltodextrin in einer Menge von 70 bis 90 Gew.-% des Aromas.
und
(b) einen zweiten Behälter, der eine zweite Zusammensetzung enthält, die 5 bis 15 g einer organischen Säurekomponente pro Liter herzustellender Lösung umfasst.

17. Kit nach Anspruch 16, umfassend zwei Beutel mit den Komponenten:
BEUTEL 1
| | |
|---|---|
| PEG 3350: | 100,000 g |
| Natriumsulfat: | 7,500 g |
| Natriumchlorid: | 2,691 g |
| Kaliumchlorid: | 1,015 g |
| Aspartam: | 0,175 g |
| Acesulfam K: | 0,117 g |
| Orangenaroma: | 0,6 g |
BEUTEL 2
| | |
|---|---|
| Ascorbinsäure: | 4,700 g |
| Natriumascorbat: | 5,900 g |

18. Einzeldosiszusammensetzung zum Mischen mit Wasser, wobei die Zusammensetzung gegebenenfalls in zwei oder mehr Teilen vorliegt und die folgenden Komponenten umfasst:
(a) 85 bis 115 g Polyethylenglykol (PEG) mit einem durchschnittlichen Molekulargewicht von 2500 bis 4500;
(b) 6 bis 9 g Natriumsulfat;
(c) 2 bis 3 g Natriumchlorid;
(d) 0,5 bis 1,5 g Kaliumchlorid;
(e) 5 bis 15 g einer organischen Säurekomponente; und
(f) Orangenaroma, umfassend Maltodextrin in einer Menge von 70 bis 90 Gew.-% des Aromas.

19. Lösung nach Anspruch 14, Kit nach Anspruch 15, 16 oder 17 oder Einzeldosis nach Anspruch 18, **dadurch gekennzeichnet, dass** die Lösung, das Kit oder die Einzeldosis eines oder mehrere der Merkmale der in einem oder mehreren der Ansprüche 1 bis 13 angegebenen Zusammensetzung aufweist.

20. Orale Lösung zur Reinigung des Darms umfassend:
(a) 85 bis 115 g/l Polyethylenglykol (PEG) mit einem durchschnittlichen Molekulargewicht von 2500 bis 4500;
(b) 6 bis 9 g/l Natriumsulfat;
(c) 2 bis 3 g/l Natriumchlorid;
(d) 0,5 bis 1,5 g/l Kaliumchlorid;
(e) 5 bis 15 g/l einer organischen Säurekomponente; und
(f) Orangenaroma, umfassend Maltodextrin in einer Menge von 70 bis 90 Gew.-% des Aromas,
zur Verwendung bei einem Verfahren zur Reinigung des Darms eines Patienten, bei dem der Patienten die Lösung einnimmt.

21. Orale Lösung zur Reinigung des Darms nach Anspruch 20, bei der 1 bis 2 Liter der Lösung zur Reinigung des Darms von dem Patienten aufgenommen werden.

22. Orale Lösung zur Reinigung des Darms nach Anspruch 21, bei der die Lösung zur Reinigung des Darms als geteilte Behandlung eingenommen wird, wobei 1 Liter Reinigungslösung am Abend vor einer klinischen Untersuchung oder eines klinischen Eingriffs und 1 Liter am frühen Morgen des Tags einer Untersuchung oder eines Eingriffs genommen werden.

23. Orale Lösung zur Reinigung des Darms nach Anspruch 20 oder 21, bei der die Lösung zur Reinigung des Darms am Abend vor einer Untersuchung oder einem Eingriff eingenommen wird.

24. Orale Lösung zur Reinigung des Darms nach einem der Ansprüche 20 bis 23, bei der der Patient zusätzlich einen weiteren Liter klare Flüssigkeit aufnimmt.

25. Orale Lösung zur Reinigung des Darms nach Anspruch 20, bei der am Abend vor einer Koloskopie oder einer anderen Untersuchung oder einem anderen Eingriff ein erster Liter Reinigungslösung über ungefähr eine Stunde eingenommen wird und dann 0,5 Liter klare Flüssigkeit und am Morgen der Koloskopie oder einer anderen Untersuchung oder einem anderen Eingriff ein zweiter Liter Reinigungslösung über ungefähr eine Stunde eingenommen wird, gefolgt von 0,5 Litern klarer Flüssigkeit.

26. Orale Lösung zur Reinigung des Darms nach Anspruch 20, bei der am Abend vor einer Koloskopie oder einer anderen Untersuchung oder einem anderen Eingriff ein erster Liter Reinigungslösung über ungefähr eine Stunde eingenommen wird und dann, ungefähr 1,5 Stunden später, ein zweiter Liter Reinigungslösung über ungefähr eine Stunde eingenommen wird, wobei während des Abends zusätzlich 1 Liter zusätzliche klare Flüssigkeit aufgenommen wird.

27. Zusammensetzung nach einem der Ansprüche 1 bis 13 oder Lösung nach Anspruch 14 oder 19 zur Verwendung als Medikament.

## Revendications

1. Une composition sèche à mélanger avec de l'eau, **caractérisée en ce que** la composition se présente éventuellement en deux ou plusieurs parties et comprend, par litre de solution à préparer, les composants suivants :
(a) 85 à 115 g de polyéthylène glycol (PEG) ayant une masse moléculaire moyenne de 2500 à 4500 ;
(b) 6 à 9 g de sulfate de sodium ;
(c) 2 à 3 g de chlorure de sodium ;
(d) 0,5 à 1,5 g de chlorure de potassium ;
(e) 5 à 15 g d'un composant acide organique ; et
(f) un aromatisant à l'orange comprenant une quantité de maltodextrine de 70 à 90 % en masse de l'aromatisant.

2. Une composition selon la revendication 1, dans laquelle l'aromatisant à l'orange comprend une quantité de maltodextrine de 70 à 80 % en masse de l'aromatisant.

3. Une composition selon la revendication 2, dans laquelle la maltodextrine est de la maltodextrine de maïs.

4. Une composition selon l'une quelconque des revendications 1 à 3, dans laquelle l'aromatisant à l'orange comprend une quantité de dextrose de 15 à 25 % en masse de l'aromatisant.

5. Une composition selon l'une quelconque des revendications 1 à 4, dans laquelle l'aromatisant à l'orange est exempt de gomme.

6. Une composition selon l'une quelconque des revendications 1 à 5, dans laquelle l'aromatisant à l'orange comprend moins de 2 % en masse de composants organiques autres que des préparations et composants aromatisants.

7. Une composition selon l'une quelconque des revendications 1 à 6, dans laquelle l'aromatisant à l'orange est présent dans une quantité comprise de 0,1 à 0,9 g par litre de solution à préparer.

8. Une composition selon l'une quelconque des revendications 1 à 7, dans laquelle la composition comprend un édulcorant, par exemple l'aspartame, l'acésulfame de potassium (acésulfame K), ou les deux.

9. Une composition selon la revendication 8, dans laquelle l'aspartame est présent dans une quantité de 0,175 g par litre de solution à préparer.

10. Une composition selon la revendication 8 ou la revendication 9, dans laquelle l'acésulfame de potassium est présent dans une quantité de 0,177 g par litre de solution à préparer.

11. Une composition selon l'une quelconque des revendications 1 à 10, comprenant de l'acide ascorbique et de l'ascorbate de sodium.

12. Une composition selon l'une quelconque des revendications 1 à 11, laquelle composition est exempte de bicarbonate de sodium.

13. Une composition selon l'une quelconque des revendications 1 à 12, comprenant, par litre de solution à préparer, les composants suivants :
(a) 100 g de polyéthylène glycol (PEG) 3350 ;
(b) 7,5 g de sulfate de sodium ;
(c) 2,691 g de chlorure de sodium ;
(d) 1,015 g de chlorure de potassium ;
(e1) 4,7 g d'acide ascorbique ;
(e2) 5,9 g d'ascorbate de sodium ;
(f) 0,6 g d'aromatisant à l'orange.
(g1) 0,175 g d'aspartame ; et
(g2) 0,117 g d'acésulfame K.

14. Une solution aqueuse comprenant de l'eau et :
(a) 85 à 115 g/l de polyéthylène glycol (PEG) ayant une masse moléculaire moyenne de 2500 à 4500 ;
(b) 6 à 9 g/l de sulfate de sodium ;
(c) 2 à 3 g/l de chlorure de sodium ;
(d) 0,5 à 1,5 g/l de chlorure de potassium ;
(e) 5 à 15 g/l d'un composant acide organique ; et
(f) un aromatisant à l'orange comprenant une quantité de maltodextrine de 70 à 90 % en masse de l'aromatisant.

15. Un kit qui comprend les composants d'une composition selon l'une quelconque des revendications 1 à 13 en deux ou plusieurs parties.

16. Un kit selon la revendication 15, comprenant :
(a) un premier récipient contenant une première composition, ladite première composition comprenant par litre de solution à préparer :
(i) 85 à 115 g de polyéthylène glycol (PEG) ayant une masse moléculaire moyenne de 2500 à 4500 ;
(ii) 6 à 9 g de sulfate de sodium ;
(iii) 2 à 3 g de chlorure de sodium ;
(iv) 0,5 à 1,5 g de chlorure de potassium ; et
(v) un aromatisant à l'orange comprenant une quantité de maltodextrine de 70 à 90 % en masse de l'aromatisant ;
et
(b) un second récipient contenant une deuxième composition comprenant 5 à 15 g d'un composant acide organique par litre de solution à préparer.

17. Un kit selon la revendication 16, comprenant deux sachets comportant les composants :
SACHET 1
| | |
|---|---|
| PEG 3350 : | 100,000 g |
| Sulfate de sodium : | 7,500 g |
| Chlorure de sodium : | 2,691 g |
| Chlorure de potassium : | 1,015 g |
| Aspartame : | 0,175 g |
| Acésulfame K : | 0,117 g |
| Aromatisant à l'orange : | 0,6 g |
SACHET 2
| | |
|---|---|
| Acide ascorbique : | 4,700 g |
| Ascorbate de sodium : | 5,900 g |

18. Une composition en dose unitaire à mélanger avec de l'eau, dans laquelle la composition se présente éventuellement en deux ou plusieurs parties et comprend les composants suivants :
(a) 85 à 115 g de polyéthylène glycol (PEG) ayant une masse moléculaire moyenne de 2500 à 4500 ;
(b) 6 à 9 g de sulfate de sodium ;
(c) 2 à 3 g de chlorure de sodium ;
(d) 0,5 à 1,5 g de chlorure de potassium ;
(e) 5 à 15 g d'un composant acide organique ; et
(f) un aromatisant à l'orange comprenant une quantité de maltodextrine de 70 à 90 % en masse de l'aromatisant.

19. Une solution selon la revendication 14, un kit selon la revendication 15, 16 ou 17, ou une dose unitaire selon la revendication 18, **caractérisé en ce que** la solution, le kit ou la dose unitaire a une ou plusieurs des caractéristiques d'une composition citée dans une ou plusieurs des revendications 1 à 13.

20. Une solution orale de nettoyage du côlon comprenant :
(a) 85 à 115 g/l de polyéthylène glycol (PEG) ayant une masse moléculaire moyenne de 2500 à 4500 ;
(b) 6 à 9 g/l de sulfate de sodium ;
(c) 2 à 3 g/l de chlorure de sodium ;
(d) 0,5 à 1,5 g/l de chlorure de potassium ;
(e) 5 à 15 g/l d'un composant acide organique ; et
(f) un aromatisant à l'orange comprenant une quantité de maltodextrine de 70 à 90 % en masse de l'aromatisant,
pour utilisation dans une méthode de nettoyage du côlon d'un sujet, dans laquelle le sujet ingère la solution.

21. Une solution orale de nettoyage du côlon selon la revendication 20, dans laquelle 1 à 2 litres de la solution de nettoyage du côlon sont ingérés par le sujet.

22. Une solution orale de nettoyage du côlon selon la revendication 21, dans laquelle la solution de nettoyage du côlon est un traitement à prendre en deux fois, 1 litre de solution de nettoyage étant pris la veille au soir d'une procédure ou d'un examen clinique et 1 litre étant pris tôt le matin du jour de l'examen ou de la procédure.

23. Une solution orale de nettoyage du côlon selon la revendication 20 ou 21, dans laquelle la solution de nettoyage du côlon est prise la veille au soir d'un examen ou d'une procédure.

24. Une solution orale de nettoyage du côlon selon l'une quelconque des revendications 20 à 23, dans laquelle le sujet ingère en outre un litre de liquide clair.

25. Une solution orale de nettoyage du côlon selon la revendication 20, dans laquelle, la veille au soir d'une coloscopie ou d'un autre examen ou procédure, un premier litre de solution de nettoyage est ingéré sur environ une heure, puis 0,5 litre de liquide clair est ingéré, et, le matin de la coloscopie ou de l'autre examen ou procédure, un second litre de solution de nettoyage est ingéré sur environ une heure, suivi de 0,5 litre de liquide clair.

26. Une solution orale de nettoyage du côlon selon la revendication 20, dans laquelle, la veille au soir d'une coloscopie ou d'un autre examen ou procédure, un premier litre de solution de nettoyage est ingéré sur environ une heure, puis, environ une heure et demie plus tard, un second litre de solution de nettoyage est ingéré sur environ une heure, 1 litre de liquide clair étant en outre ingéré durant la soirée.

27. Une composition selon l'une quelconque des revendications 1 à 13, ou une solution selon la revendication 14 ou 19, pour utilisation comme médicament.
